(19) Europäisches Patentamt
European Patent Office
Office européen des brevets

(11) Publication number: 0 228 075 A2

# (12) EUROPEAN PATENT APPLICATION

(21) Application number: 86117978.6

(22) Date of filing: 23.12.86

(51) Int. Cl.⁴: C 12 Q 1/68 // C07H 21/00

(30) Priority: 03.01.86 US 815974
28.03.86 US 845221

(43) Date of publication of application: 08.07.87 Bulletin 87/28

(84) Designated Contracting States: DE FR GB GR IT SE

(71) Applicant: Molecular Diagnostics, Inc., 400 Morgan Lane, West Haven, CT.06516 (US)

(72) Inventor: Dattagupta, Nanibhushan, 470 Prospect Street, New Haven, CT 06511 (US)
Inventor: Rabin, Daniel, 24 Stone Street, Branford, CT 06405 (US)

(74) Representative: Jesse, Ralf-Rüdiger, Dr. et al, Bayer AG Konzernverwaltung RP Patentabteilung, D-5090 Leverkusen 1 Bayerwerk (DE)

## (54) Eucaryotic genomic dna dot-blot hybridization method.

(57) A dot-blot hybridization method and test kit for detecting normal and mutated genes in unfractionated eucaryotic genomic DNA. Unfractionated single stranded sample DNA is immobilized in a limited zone on a solid support sheet and hybridized with an oligonucleotide probe complementary to the sequence of interest. After stringency washing to remove nonspecifically hybridized probe, the dot-blot zone is examined for the presence of hybridized probe, usually by detection of a label attached to the probe. Hybridization conditions are selected to provide a level of detectable hybridized probe in the dot-blot zone which is readily distinguishable from any background signal associated with the surrounding area on the support sheet. The method eliminates the need for substantial purification and fragmentation of sample DNA required by prior art techniques.

EP 0 228 075 A2

# EUCARYOTIC GENOMIC DNA DOT-BLOT HYBRIDIZATION METHOD

## BACKGROUND OF THE INVENTION

This invention relates to the detection of normal and mutated base sequences or genes in eucaryotic genomic DNA. In particular, the invention concerns such detection involving nucleic acid hybridization of the sequence of interest with an oligonucleotide probe.

Oligonucleotide probes serve as the basis of a powerful method for detecting the presence of specific DNA sequences in a mixed DNA population. Generally, the test DNA is immobilized on a solid support, often nitrocellulose. The immobilized DNA is then exposed to labeled oligonucleotide in a hybridization mixture. The immobilized phase is then washed under stringent conditions of buffer and temperature such that specific binding is maintained, but that nonspecific binding is destabilized and lost. This process forms the basis of the Southern blotting procedure (Southern, E.M., (1975), J. Mol. Biol., 98, 503-517). A shortcut variation of this procedure eliminates the gel electrophoresis step for separation of the test DNA fragments. Because it is not separated, DNA tested in the so-called "dot-blot" procedure does not have to be cut with restriction endonuclease. Elimination of these two steps shortens and simplifies the procedure considerably when a "yes/no" answer is sufficient: Is DNA of a given sequence present or not? Besides the time and effort saving in the dot-blot procedure, one can test at least ten times as

many samples per unit area of the solid support as with the Southern and derivative procedures.

Dot-Blot and related procedures are common in procaryotic systems. But because of the thousand-fold higher complexity of eucaryotic genomes, a restriction/separation scheme has heretofore been necessary for single copy gene detection. Thus far, the simple format and ease of handling of the dot-blot procedure has only been possible in procaryotic systems (e.g., Grunstein, M. and Hogness, D.C., (1975), Proc. Natl. Acad. Sci. USA, 42, 3961-3965).

The introduction of synthetic oligonucleotides has revolutionized the field of DNA hybridization and has permitted cloning of genes whose protein sequence is only partially known. In the area of human genetic analysis, it has permitted the generation of probes of arbitrary length (routinely up to sizes of 30-40 nucleotides) to cover any region of any gene whose sequence is known. Wallace and co-workers (Conner, B.J., Reyes, A.A., Morin, C., Itakura, K., Teplitz, R.L., and Wallace, R.B., (1983), Proc. Natl. Acad. Sci. USA, 80, 278-282) have studied the sickle cell mutation in the beta-globin gene and determined that optimal discrimination between normal and sickle sequences can be made with probes of 19 nucleotides with the mutant base in position 13. However, the method has the significant disadvantages of requiring restriction endonuclease digestion and gel electrophoresis separation of the resulting DNA fragment mixture. Wallace et al use these elaborate DNA fractionation and separation techniques in order to isolate small DNA fragments for oligonucleotide hybridization. These workers and others have observed high background binding of the oligonucleotide probe to high molecular weight DNA which would prevent detection of specific hybridization in the unfractionated DNA mixture.

Wood et al (Wood, W.I., Gitschier, J., Lasky, L.A., and Lawn, R.M., (1985), Proc. Natl. Acad. Sci. USA, 82, 1585-1588) explored the use of tetramethylammonium chloride (TMAC) in a stringency washing medium. This salt reduces the sequence dependence of oligonucleotide hybridization, permitting hybridization with probes of mixed sequence and stringency washing that discriminates only on the basis of extent of base pairing. The authors mention incidentally that they have used oligonucleotide hybridization to detect a single base polymorphism in the factor VIII gene. This method is identical to that of Conner et al mentioned above with the addition of a transfer step. This adds to the complexity of the Conner et al method. The introduction of the TMAC wash, however, seems to have eliminated nonspecific background that is usually observed in the high molecular weight range. Thus, the prior art methods for detecting normal and mutated gene sequences in eucaryotic genomic DNA, particularly human DNA, by oligonucleotide hybridization require extensive and complicated DNA purification, involving DNA restriction digestion and gel electrophoresis, in order to provide a detectable, specific result.

There are presently many methods of labeling oligonucleotides known in the art. Photochemical labeling has been described in European Patent Publications 131 830 and 156,287. A general scheme for coupling a nucleic acid, exemplified by double-stranded DNA, to a label such as a hapten or enzyme is as follows:

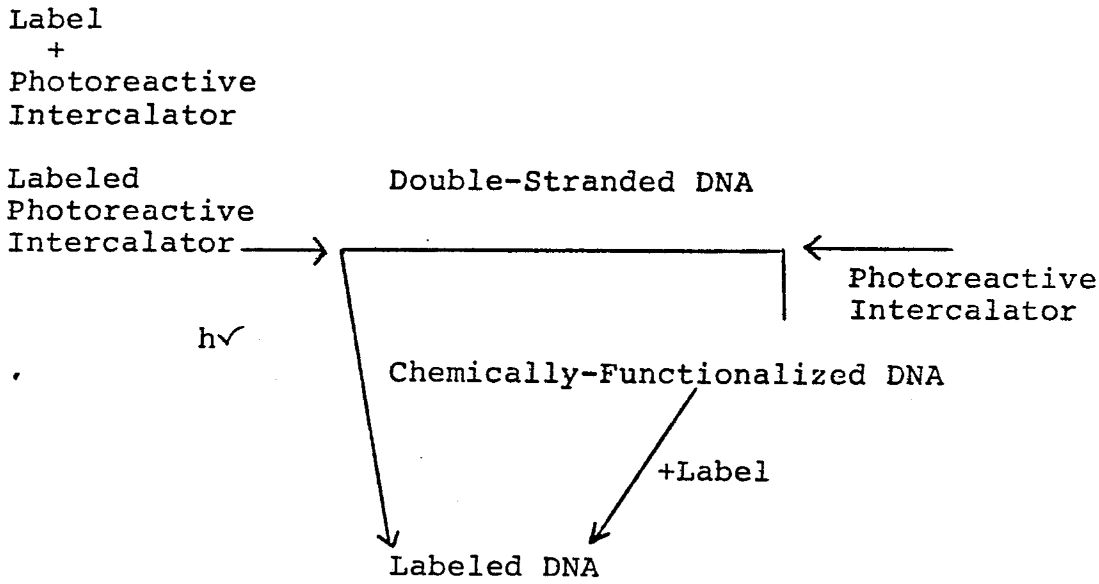


Heretofore there has been no disclosure of photochemical labeling with an oligonucleotide probe. It is known, however, that if an oligonucleotide is modified in the hybridizing region, the hybridization parameters can be different than the intact oligonucleotide.

The background problems evident from the prior art studies involving oligonucleotide hybridization have accordingly severly limited attempts to simplify the procedure. Thus, despite the ever increasing need to eliminate cumbersome and time consuming steps in hybridization analysis in order to facilitate practical application of the technique, such as in clinical laboratories, oligonucleotide hybridization has continued to be burdened by elaborate sample handling and preparative steps. No attempt has apparently been made to perform a dot-blot hybridization using oligonucleotide probes to detect sequences in unfractionated eucaryotic genomic DNA despite the clear advantages of such a technique.

## SUMMARY OF THE INVENTION

It has now been surprisingly found that nucleotide sequences in eucaryotic genomic DNA can be detected by oligonucleotide dot-blot hybridization. The method is applicable to the detection of normal and mutated gene sequences, particularly those sites at which point mutations occur. The use of oligonucleotide probes enable the assay method to distinguish known base sequences from sequences in which a few or even a single base is different. The method finds particular use in the genetic analysis of human genomic DNA. The dot-blot procedure eliminates the need for fragmentation of the sample DNA and gel electrophoresis to separate fragments containing the sequence of interest from background DNA.

The present method involves immobilization of DNA from the test sample in single stranded form in a limited zone or area (the dot-blot) of a solid support sheet such as nitrocellulose. After treatment to substantially block unoccupied nucleic acid binding sites, the support sheet is incubated with a solution of an oligonucleotide probe, preferably comprising a detectable label, having a base sequence exactly complementary to the base sequence of interest. The incubation is accomplished under hybridizing conditions favoring the hybridization of probe to the sequence of interest. After removing the probe solution, the support is washed at a stringency sufficient to remove substantially all probe that had hybridized to sequences other than the exactly complementary sequence of interest. The presence of hybridized probe in the dot-blot is then detected and indicates the presence of the sequence of interest in the sample of human genomic DNA. Where the probe is labeled, the presence of hybridized probe in the dot-blot is readily determined by detecting the signal or response of the label.

The conditions of hybridization are selected such that after stringency washing a level of detectable hybridized probe remains in the dot-blot which is readily distinguishable from any background binding of probe to the surrounding support sheet. Since the method is based on the dot-blot technique, the need for substantial purification and fragmentation of sample DNA required by the prior art techniques is eliminated. The sample can be simply treated, such as with base followed by neutralization, to denature genomic DNA and applied to the support sheet. An alternative is to immobilize sample DNA directly from whole cells collected such as by filtration onto the sheet, followed by denaturation in situ on the sheet. As a result, the normally labor intensive and time consuming DNA isolation can be reduced to a few manipulations of the solid support and allows multiple samples to be analyzed on a single sheet.

In the analysis of human genomic DNA for the presence of the normal or a mutated gene sequence of interest, it is preferred to perform simultaneous hybridizations for such respective sequences. This is very readily accomplished using the present dot-blot technique by applying portions of the sample to separate first and second zones on the support sheet. A pair of oligonucleotide probes are used; one exactly complementary to the normal sequence and the other exactly complementary to the mutated sequence. Before incubation of the sheet with the hybridization solution, the support sheet is cut to form two sections, one comprising the first zone and the other the second zone. Each of the pair of probes is then contacted with one of the cut sections and the method continued as described above with respect to both of the cut sections. Analysis of the presence of hybridized probe in the two zones cut from the support sheet will indicate the presence of either or both of the sequences

of interest in the sample.

Applicants have discovered a probe system where the hybridizing region of the oligonucleotide is not appreciably modified whereas, an extra region that is non-complementary to the target DNA specifically carries the label. In order to accomplish this, applicants have discovered a probe system comprising two synthetic oligonucleotides, one of which carries the hybridizing region specific for the detection of a particular nucleic acid sequence, for example, a mutation, for example, a point mutation, or a frameshift or a deletion and nucleotide residues adjacent to such hybridizing region. Such nucleotide residues numbering between 5 and 10,000 nucleotide residues. The nucleotide residues adjacent to the hybridizing region does not participate in the hybridization. The other oligonucleotide is complementary to the surrounding nucleotide residues of the specific strand.

When these two oligonucleotides are mixed together they form a double stranded region and a single stranded hybridizable region in one hybridizing molecule. A label is attached to the double stranded region.

The double stranded region can be modified with an intercalating drug. This can be done by using photochemically active intercalating compounds, for example, aminomethyltrioxsalen. The amine can then be used to attach the desired label. The desired label can be a small molecule like biotin or other haptens, immunogens, or a fluorophore like fluoresceine. It can also be an enzyme, e.g., horseradish peroxidase.

The reaction can be carried out in two different ways, namely as follows:

(1) the photoreactive intercalator, e.g., aminomethyltrioxsalen can be reacted with the label and the final product can be photochemically reacted with the mixed probe;

(2) photoreactive intercalator, e.g., aminomethyltrioxsalen can be photochemically linked to the DNA first and then the product can be reacted with the label.

To produce specific and efficient photochemical products, it is desirable that the nucleic acid component and the photoreactive intercalator compound be allowed to react in the dark in a specific manner.

For photochemical coupling to nucleic acids, aminomethyl psoralen, aminomethyl angelicin and amino alkyl ethidium or methidium azides are particularly useful compounds. They bind to double-stranded DNA and only the complex produces photoadduct. Angelicin derivatives are superior to psoralen compounds for monoadduct formation. If a single-stranded probe is covalently attached to some extra double-stranded DNA, use of phenanthridium and psoralen compounds is desirable since these compounds interact specifically to double-stranded DNA in the dark. The chemistry for the synthesis of the coupled reagents to modify nucleic acids for labelling, described more fully hereinbelow, is similar for all cases.

The nucleic acid component can be DNA or RNA or relatively short oligomers.

The nucleic acid-binding ligands of the present invention used to link the nucleic acid component to the label can be any suitable photoreactive form of known nucleic acid-binding ligands. Particularly preferred nucleic acid-binding ligands are intercalator compounds such as the furocoumarins, e.g., angelicin (isopsoralen) or psoralen or derivatives thereof which photochemically will react with nucleic acids, e.g., 4'-aminomethyl-4,5'-dimethyl angelicin, 4'-aminomethyltrioxsalen (4'-aminomethyl-4,5',8-trimethyl-psoralen, 3-carboxy-5- or -8-amino- or - hydroxy-psoralen, as well as mono- or bis-azido aminoalkyl methidium or ethidium

compounds. Photoreactive forms of a variety of other intercalating agents can also be used as exemplified in the following table:

| Intercalator Classes and Representative Compounds | Literature References |
|---|---|
| A. Acridine dyes | Lerman, J. Mol. Biol. 3:18(1961); Bloomfield et al, "Physical Chemistry of Nucleic Acids", Chapter 7, pp. 429-476, Harper and Rowe, NY(1974) |
| proflavin, acridine orange, quinacrine, acriflavine | Miller et al, Biopolymers 19:2091(1980) |
| B. Phenanthridines | Bloomfield et al, supra |
| | Miller et al, supra |
| ethidium | |
| coralyne | Wilson et al, J. Med. Chem. 19:1261(1976) |
| ellipticine, ellipticine cation and derivatives | Festy et al, FEBS Letters 17:321(1971); Kohn et al, Cancer Res. 35:71(1976); LePecq et al, PNAS (USA)71: 5078(1974); Pelaprat et al, J. Med. Chem. 23:1330(1980) |
| C. Phenazines<br>5-methylphenazine cation | Bloomfield et al, supra |
| D. Phenothiazines<br>chlopromazine | ibid |
| E. Quinolines<br>chloroquine<br>quinine | ibid |
| F. Aflatoxin | ibid |
| G. Polycyclic hydrocarbons and their oxirane derivatives | ibid |

| | |
|---|---|
| 3,4-benzpyrene benzopyrene diol epoxide, 1-pyrenyl-oxirane | Yang et al, Biochem. Biophys. Res. Comm. 82:929(1978) |
| benzanthracene-5,6-oxide | Amea et al, Science 176:47(1972) |
| H. Actinomycins actinomycin D | Bloomfield et al, supra |
| I. Anthracyclinones B-rhodomycin A daunamycin | ibid |
| J. Thiaxanthenones miracil D | ibid |
| K. Anthramycin | ibid |
| L. Mitomycin | Ogawa et al, Nucl. Acids Res., Spec. Publ. 3:79(1977); Akhtar et al, Can. J. Chem. 53:2891(2975) |
| M. Platinium Complexes | Lippard, Accts. Chem. Res. 11:211(1978) |
| N. Polyintercalators echinomycin | Waring et al, Nature 252:653(1974); Wakelin, Biochem. J. 157:721(1976) |
| quinomycin triostin BBM928A tandem | Lee et al, Biochem. J. 173:115(1978): Huang et al, Biochem. 19: 5537(1980): Viswamitra et al, Nature 289: 817(1981) |
| diacridines | LePecq et al, PNAS (USA)72:2915(1975): Carrellakis et al, Biochim. Biophys. Acta 418:277(1976); Wakelin et al, Biochem 17:5057(1978); Wakelin et al, FEBS Lett. 104:261(1979); Capelle et al, Biochem. 18:3354 |

| | |
|---|---|
| | (1979); Wright et al, Biochem. 19:5825(1980); Bernier et al, Biochem. J. 199:479 (1981); King et al, Biochem. 21:4982 (1982) |
| ethidium dimer | Gaugain et al, Biochem. 17:5078(1978); Kuhlman et al, Nucl. Acids Res. 5:2629(1978); Marlcovits et al, Anal. Biochem. 94:259(1979): Dervan et al, JACS 100:1968(1978); ibid 101:3664(1979). |
| ellipticene dimers and analogs | Debarre et al, Compt. Rend. Ser. D. 284: 81(1977); Pelaprat et al, J. Med. Chem. 23:1336(1980) |
| heterodimers | Cain et al, J. Med. Chem. 21:658(1978); Gaugain et al, Biochem. 17:5078(1978) |
| trimers | Hansen et al, JCS Chem. Comm. 162(1983); Atnell et al, JACS 105:2913(1983) |
| O. Norphillin A | Loun et al, JACS 104: 3213(1982) |
| P. Fluorenes and fluorenones | Bloomfield et al, supra |
| fluorenodiamines | Witkowski et al, Wiss. Beitr.-Martin-Luther-Univ. Halle Wittenberg, 11(1981) |
| Q. Furocoumarins | |
| angelicin | Venema et al, MGG, Mol. Gen. Genet. 179;1 (1980) |
| 4,5'-dimethylangelicin | Vedaldi et al, Chem.-Biol. Interact. 36: 275(1981) |

| | |
|---|---|
| psoralen | Marciani et al, Z. Naturforsch B 27(2): 196(1972) |
| 8-methoxypsoralen | Belognzov et al, Mutat. Res. 84:11(1981); Scott et al, Photochem. Photobiol. 34:63(1981) |
| 5-aminomethyl-8-methoxypsoralen | Hansen et al, Tet. Lett. 22:1847(1981) |
| 4,5,8-trimethylpsoralen | Ben-Hur et al, Biochem. Biophys. Acta 331:181(1973) |
| 4'-aminomethyl-4,5,8-trimethylpsoralen | Issacs et al, Biochem. 16:1058(1977) |
| xanthotoxin | Hradecma et al, Acta Virol. (Engl. Ed.) 26: 305(1982) |
| khellin | Beaumont et al, Biochim. Biophys. Acta 608:1829(1980) |
| R. Benzodipyrones | Murx et al, J. Het. Chem. 12:417(1975); Horter et al, Photochem. Photobiol. 20: 407(1974) |
| S. Monostral Fast Blue | Juarranz et al, Acta Histochem. 70:130 (1982) |

Particularly useful photoreactive forms of such intercalating agents are the azidointercalators. Their reactive nitrenes are readily generated at long wavelength ultraviolet or visible light and the nitrenes of arylazides prefer insertion reactions over their rearrangement products [see White et al, Methods in Enzymol., 46, 644(1977)]. Representative azidointercalators are 3-azidoacridine, 9-azidoacridine, ethidium monoazide, ethidium diazide, ethidium dimer azide [Mitchell et al, JACS, 104, 4265(1982)], 4-azido-7-chloroquinoline, and 2-azidofluorene. Other

useful photoreactable intercalators are the furocoumarins which form [2+2] cycloadducts with pyrimidine residues. Alkylating agents can also be used such as bis-chloroethylamines and epoxides or aziridines, e.g., aflatoxins, polycyclic hydrocarbon epoxides, mitomycin, and norphillin A.

The label which is linked to the nucleic acid component according to the present invention can be any chemical group or residue having a detectable physical or chemical property. The label will bear a functional chemical group to enable it to be chemically linked to the intercalator compound.

## BRIEF DESCRIPTION OF THE DRAWINGS

Fig. 1 is a photograph of dot-blots resulting from radiolabeled probe hybridizations according to the present invention applied to the detection of normal and sickle cell mutated forms of the human beta-globin gene.

Fig. 2 is a photograph of dot-blots according to Example 2 described hereinbelow.

## DESCRIPTION OF THE PREFERRED EMBODIMENTS

### Test Sample

The present method can be applied to the detection of eucaryotic genomic DNA sequences in essentially any specimen or medium which can be used directly or which can be processed to yield a suitable test sample for application to the support sheet for immobilization of sample DNA. Such specimens and media include nucleic acids or cells from liquid, semi-solid, or solid samples of medical, veterinary, environmental, nutritional, or industrial significance. Human and animal specimens and

body fluids include those containing nucleated cells such as urine, blood (whole blood, serum or plasma), milk, cerebrospinal fluid, tissue sections, and the like.

Any eucaryotic genomic DNA sequence whose base composition is known can be determined according to the present method. In the case of humans and animals, various genetic disorders can be detected, such as sickle cell, alpha-1-protease inhibitor mutations, thallasemias, and hemoglobin C, where the normal gene sequence and its mutated form or forms are known. The mutation can take any form that can be distinguished from the normal sequence by oligonucleotide hybridization, including deletions, insertions, inversions, frameshift, base modifications, and particularly point mutations where a single base is altered.

The present procedure can also be used to simplify restriction fragment length polymorphism (RFLP) analysis in the case where the sequence of interest is known. For example, if restriction nuclease susceptibility is affected by a point mutation, the RFLP can be used to diagnose the mutation. The dot-blot format can be used either instead of the RFLP or in conjunction with the restriction nuclease: first digesting the DNA, then using dot-blot hybridization to assay whether the sequence has been cut by the enzyme. Even in the case where there is only a correlation between a sequence and a trait this procedure would be applicable. In this scheme it is not necessary for the oligonucleotide probe recognition sequence to be within or contiguous with the gene of interest; only that a correlation can be established.

The following table, taken from "The New Genetics and Clinical Practice" by D.J. Wetherall (1982), The Nuffield Provincial Hospitals Trust, lists some genetic probes of potential medical interest.

Specific

Globin gene clusters α, β, γ, δ, ε,
Growth hormone
Chorionic somatomammotrophin
Chorionic gonadotrophin
Prolactin
Insulin
Interferons
α chain of type I collagen
HLA β
Histone (several)
Immunoglobulin genes (heavy chain, κ and λ light chains)
Gastrin
Ribosomal RNA
Melanocyte stimulating hormone (α, β, γ)
Corticotrophin
β Lipoprotein
β Endorphin
Actin
$\alpha_1$ Antitrypsin
Albumin
Glucose-6-phosphate dehydrogenase
Pre-parathyroid hormone
$\beta_1$ Microglobulin
α-Tubulin
Factors VII and IX
Antithrombin 3
Several components of complement pathway

Non-specific

Chromosome 2,3,5,6,11,14,20,22 and X

Sources include Antonarakis, S.E., Phillips, J.A., and Kazazian, H.H., (1982), J. Pediat., 100, 845-856.

Where the presence of a normal gene or a specific mutation of the gene needs to be positively established, hybridizations will be performed on separate portions of the test sample using probes respectively complementary to the normal and mutated sequence. The present method provides a convenient means for performing such a side-by-side analysis and for performing multiple such analyses on a batch of test samples. Of course, in a situation where multiple known mutations of the normal gene sequence are of significance, multiple hybridizations can be conducted using the present method wherein additional dot-blot zones are applied to the support sheet and separate hybridizations with the appropriate respective probes are performed.

The example below uses purified DNA. This was for purposes of development as well as comparison with prior art techniques which require purified DNA. One of the advantages of the dot-blot technique is that there is considerable room for simplification of sample preparation: Crude DNA or even whole cells (e.g., Brandsma, U. and Miller, G., (1980), Proc. Nat'l. Acad. Sci. USA, 77, 6851-6855) can be applied to the filter for hybridization testing. Crude DNA preparations may even be better than purified DNA because the proteins and other cellular components could help the initial sticking of the test DNA to the solid support.

## Dot-Blotting Technique

Essentially any procedure can be used to make the dot-blots on the support sheet provided that it results in immobilization of DNA from the test sample in single stranded form in a limited zone of the sheet. It is necessary that a sufficient amount of the sample DNA be rendered single stranded and immobilized on the support to

enable detectable hybridization with the oligonucleotide probe to occur.

Several procedures for obtaining immobilized single stranded DNA are preferred. In one such procedure, the test sample as a liquid mixture is treated in known manners to denature DNA therein and the resulting solution applied to the support sheet. Using conventional dot-blot apparatus, residual liquid is then removed by vacuum through the sheet. Denaturation of sample DNA can, for example, be accomplished by addition of base, such as 0.1 to 1.0 molar sodium hydroxide, followed by neutralization prior to application to the support sheet. Heat treatment, organic solvents, and other denaturants can also be used for this purpose. Alternatively, the liquid test sample can be applied directly to the support and the DNA denatured in situ. Conventional techniques dictate laying the test fulter on filter paper saturated with a) 0.5 M NaOH, b) neutral pH 1.0 M Tris, then c) neutral pH 1.0 M Tris plus 1-3M NaCl. This procedure, however, places limitations on the sample volume; typically less than 10 µl, preferably 1-2 µl. The dot-blot procedure can accomodate sample volumes up to several hundred µl. Strong base treatment tends to make nitrocellulose paper brittle, which favors the dot-blot procedure.

While conventional dot-blotting apparatus can be very conveniently used to apply the sample to the support sheet, essentially any technique can be used which effectively localizes the test sample in a definable area or zone on the support. Similarly, the shape and size of such zone, which is herein generically referred to as a dot-blot, is not critical.

A variety of support materials are known in the art to be capable of immobilizing single stranded DNA. Such materials include commercially available, commonly used nitrocellulose paper, various nylon sheets, e.g.,

Gene-Screen from duPont, Wilmington, Delaware, U.S.A. and ZETA-PROBE from Bio-Rad, California, U.S.A. diazobenzyloxymethyl paper, and so forth. The choice of support material is not critical. After application of the test sample to the support, and performance of any additional steps required for denaturation, the single stranded DNA adsorbed to the support is normally subjected to further treatment such as heating in vacuo or alcohol, to firmly fix the DNA to the sheet.

It is generally necessary to treat the dot-blotted support to block unoccupied nucleic acid binding sites on the sheet before allowing hybridization with the specific probe to take place. This can be achieved by any of the conventionally known methods and will vary somewhat depending on the particular nature of the support used. Normally, the sheet will be incubated for a predetermined period of time, generally between about 5 minutes and about 4 hours, preferably between about 5 and about 30 minutes, with a prehybridization solution containing chemical agents that will bind and perform the desired blocking function. Such a solution will usually comprise a high molecular weight protein such as an albumin and a nonspecific nucleic acid such as DNA from a source foreign to the test sample (conventionally salmon sperm DNA is used for this purpose). Other ingredients of the prehybridization solution can be polyvinyl pyrrolidine, Ficoll, sodium dodecyl sulfate. The prehybridization and hybridization solutions are similar, if not identical, with the addition of probe to the hybridization mix. A recent advance is the use of "Blotto", a nonfat dry milk concoction (Johnson, D.A., Gantsch, J.W., Sportsman, J.R., and Elder, J.H., (1984), Gene Anal. Techn., 1, 3-8). The prehybridization treatment will be conducted at a predetermined elevated temperature. The prehybridization temperature is generally identical to the hybridization

temperature, usually 10-20°C below the Td (dissociation temperature).

## Hybridization

The conditions of hybridization used in the present method are key to successful detection of specific hybridization with low background from probe nonspecifically bound to the support or nonspecific DNA in the dot-blot. The rate and efficiency of nucleic acid hybridizations in general depend upon the principal factors of temperature, ionic strength, presence of organic solvents, and concentration of the hybridizable nucleic acids. The present invention has established that optimum conditions of hybridization can be found to enable the use of the highly convenient dot-blot technique in detection of sequences in unfractionated eucaryotic DNA using oligonucleotide probes. Furthermore, the time of incubation of the hybridization mixture has been found to be particularly significant in obtaining easily interpretable results.

Optimal values of temperature, ionic strength, and solvent are largely empirically determined. In the example below, the Td of the specific interaction is approximately 59°C. Therefore an hybridization termperture of 50°C is used, though this is not critical. With longer probes (>50 nucleotides) where the Td is high (>80°C), formamide is often used to reduce the Td (Wood et al vide supra). Hybridization is then typically done at 68°C in 50% formamide.

With regard to the concentration of nucleic acids involved in the hybridization, it has been found that relatively lower concentrations of probe should be used than conventional for Southern blots in order to reduce background binding of use probe. The use of probe

concentrations in the hybridization solution between about 10 picomolar and about 10 nanomolar is a significant factor in the successful application of the dot-blot technique to oligonucleotide probe hybridization analysis of eucaryotic DNA sequences. In the specific example below concerning detection of the sickle cell mutation in human genomic DNA, probe concentrations between about 0.01 and about 3 nanomolar will be preferred, with optimal concentrations being between about 0.3 and about 1 nanomolar. At these concentrations, it is possible to achieve detectable signal from hybridized probe while preventing significant non-specific oligonucleotide binding to the support.

A very significant parameter of the present invention is the time of incubation of the hybridization mixture with the support. In order to limit nonspecific binding of probe to the support, the time of incubation will normally be held below about 4 hours, with the minimum incubation time being dictated by the sensitivity needed to give a detectable signal for hybridized probe. Usually, a hybridization period of at least about 10 minutes will be necessary, however, the use of highly sensitive detection methods can decrease this time. The most preferred incubation time has been found to be between about 10 minutes and 1.5 hours.

After removal of the hybridization solution from the support sheet, it is necessary to wash the support under sufficiently stringent conditions such that only hybrids of the probe and an exactly complementary base sequence remain intact. In this way, any probe that has hybridized to sequences having less than perfect homology with the probe sequence are removed from the dot-blot. The conditions of stringency to accomplish this task will vary with a) length of oligonucleotide, b) ionic strength, c)

degree and position of mismatch(es), d) bases comprising mismatch(es).

Ideally one would be able to calculate the Td of perfectly paired oligonucleotides in solution and the expected imperfect ones. In practice, however, the parameters are not known sufficiently precisely, and one measures Td experimentally.

The preparation and use of oligonucleotides as probes in DNA hybridizations are well known in the art. Such probes have defined base sequences and can vary from as few as about 10 bases up to as many as about 200 bases. Normally, useful oligonucleotide probes will have between about 14 and about 25 bases. The probe sequences can comprise deoxyribo-, ribo-, and mixed deoxyribo- and ribo-sequences. They are prepared by well known synthetic techniques such as phosphite or phosphotriester methods (Alvarado-Urbina et al (1981), Science, 214, 270-274).

## Detection of Hybrids

The present invention is not limited to particular methods or reagents for detecting hybridized probe in the dot-blot. Any convenient method can be applied. The present method enables the particular use of labeled probes since conditions have been found to limit background binding of probe to the support.

There are a variety of methods that can be used in the present invention for determining the presence of the hybridized probe. One of ordinary skill in the art can choose from any conventional means for detecting the occurrence of hybridization between the probe and the sequence to be detected and the resulting presence of the probe in the immobilized phase or its reduced presence in the reaction mixture. In general, the detection step will be based on the use of a labeled form of the probe, the

use of a probe that forms a uniquely detectable hybrid with the sequence of interest, or through secondary reactions which can only be carried out when hybridization takes place.

A particularly preferred approach to the detection step involves the use of a labeled form of the probe. The label will be a native characteristic of the oligonucleotide comprised in the probe or a substance which has a detectable physical, chemical, or electrical property. When a detectable labeling substance is introduced, it can be linked directly such as by covalent bonds to the probe or can be linked indirectly such as by incorporation of the ultimately detectable substance in a microcapsule or liposome which in turn is linked to the detection probe.

Labeling materials have been well-developed in the field of immunoassays and in general most any label useful in such methods can be applied to the present invention. Particularly useful are enzymatically active groups, such as enzymes (see Clin. Chem., (1976), 22,1232; U.S. Reissue Pat. No. 31,006, and UK Pat. 2,019,408), enzyme substrates (see U.S. Pat. No. 4,492,751), coenzymes (see U.S. Pat. Nos. 4,230,797 and 4,238,565), and enzyme inhibitors (see U.S. Pat. No. 4,234,792); fluorescers (see Clin. Chem., (1979), 25, 353); chromophores; luminescers such as chemiluminescers and bioluminescers (see U.S. Pat. No. 4,380,580); specifically bindable ligands such as biotin (see European Pat. Spec. 63,879) or a hapten (see PCT Publ. 83-2286); and radioisotopes such as $^{3}H$, $^{35}S$, $^{32}P$, $^{125}I$, and $^{14}C$. Such labels are detected on the basis of their own physical properties (e.g., fluorescers, chromophores and radioisotopes) or their reactive or binding properties (e.g., ligands, enzymes, substrates, coenzymes and inhibitors). For example, a cofactor-labeled species can be detected by adding the

enzyme (or enzymes where a cycling system is used) for which the label is a cofactor and a substrate or substrates for the enzyme. A hapten or ligand (e.g., biotin) labeled species can be detected by adding an antibody to the hapten or a protein (e.g., avidin) which binds the ligand, tagged with a detectable molecule. Such detectable molecule can be some molecule with a measurable physical property (e.g., fluorescence or absorbance) or a participant in an enzyme reaction (e.g., see above list). For example, one can use an enzyme which acts upon a substrate to generate a product with a measurable physical property. Examples of the latter include, but are not limited to, beta-galactosidase, alkaline phosphatase and peroxidase.

Methods for preparing a labeled probe used in a preferred embodiment of the present invention are readily available from the prior art. When labeling probes one will employ synthetic approaches which are effective for modifying nucleic acids without substantially interfering with the ability of the labeled probe to participate in hybridization, and will select labels which are sufficiently stable under the conditions to be used for hybridization to enable their subsequent detection.

By way of example, the following approaches can be used in labeling probes. Radiolabeled nucleotides can be incorporated into oligonucleotide probes by methods such as primer extension and terminal labeling with terminal deoxynucleotidyl transferase. A template primer pair can be chosen such that one can be extended to complete a desired sequence without extending the other of the pair. As for example, the following four pairs will produce nonadecanucleotide without the use of one of the same length as the template:

| | | | |
|---|---|---|---|
| 19A' | 5' | GCAGACTTCTCCTC | 3' |
| Pair | 3' | GAAGAGGAGTCCTC | 5' |

and

| | | | |
|---|---|---|---|
| 19S' | 5' | GCAGACTTCTCCAC | 3' |
| Pair | | GAAGAGGTGTCCTC | 5' |

Only the upper strand will be extended if deoxyadenosine triphosphate (dATP) and deoxyguanosine triphosphate (dGTP) are used as nucleoside triphosphate (NTP) substrates for DNA polymerase or reverse transcriptase reaction. This will produce 19A'/S' sequences and unextended lower strand.

Similar complementary strands can be synthesized or labeled by using pyrimidine nucleoside triphosphates instead of dATP and dGTP to produce complementary sequences. The pairs to be used are

| | | | |
|---|---|---|---|
| 19S | 5' | CTCCTGTGGAGAAGTC | 3' |
| Pair | 3' | CCTCTTCAGACG | 5' |
| 19A | 5' | CTCCTGAGGAGAAG | 3' |
| Pair | 3' | CTCCTCTTCAGACG | 5' |

Another useful set of oligonucleotide probes that can be labeled by lengthening only one member of a pair of partially complementary oligonucleotides is one that will discriminate the normal $alpha_1$-antitrypsin gene from the mutated gene that is responsible for the common form of antitrypsin deficiency. A shortage of this protease inhibitor results in emphysema and some associated disorders. The normal allele of the gene is M, and the major deficiency allele is Z; the nucleotide sequence of the gene around the position of the Z point mutation (Kidd, V.J., Wallace R.B., Itakura, K. and Woo, S.C.C., "alpha-Antitrypsin Deficiency Detention By Direct Analysis Of The Mutation In The Gene", Nature, 309, 230-234(1983))

is such that the asymmetric primer extension labeling can be applied as follows:

$^{M}$primer 5' TGCTGACCATCGACGAG
$^{M}$template AGCTGCTCTTTCCC 5'

$^{Z}$primer 5' TGCTGACCATCGACAAG
$^{Z}$template AGCTGTTCTTTCCC 5'.

Both of these can be labeled asymmetrically by providing a reverse transcriptase or Klenow polymerase reaction with only purine deoxyribonucleoside triphosphate precursors. The template remains a tetrakaidecanucleotide, while the primer can be extended from a length of 17 to either 20 by the inclusion of dATP only, or 23 by the inclusion of both dATP and dGTP in reaction mixtures.

Labeling by using these kinds of pairs is limited to sequences that can be identified as having the required proximity of a mutation and a cluster of nucleotides that will act as a template in one strand, but not the other. These may not be common. In situations where the configuration of nucleotides in a gene sequence does not allow a selection of primer and template for this labeling scheme, it is possible to synthesize a template that is blocked at its 3' end so that it cannot be extended irrespective of its sequence. Such blocking can be accomplished by incorporating a 2', 3' dideoxyribonucleotide as the ultimate 3' nucleotide during chemical synthesis of the template oligonucleotide.

A major feature of the asymmetric primer extension labeling system described in this application is that the difference in length between labeled and unlabeled oligonucleotides greatly facilitates the separation and purification of the former from the latter and from unincorporated precursors by electrophoresis or chromatography. Unexpectedly, it has also proved true

that no fractionation of the labeling reaction mixture is necessary for the preparation of a hybridization assay.

Labeled oligonucleotide is normally purified away from unlabeled oligonucleotide because the latter would be involved in hybridization and would reduce the strength of the signal resulting from labeled probe binding to sample DNA. However, the difference in length between the unlabeled template and the labeled extended primer is sufficient that under conditions that can be used for hybridization of labeled probe, the template is too short to form stable hybrids so that it does not interfere with the annealing of probe to sample DNA.

Other genetic diseases for which there is sufficient DNA sequence information to develop synthetic oligonucleotide probes are in the family of hemoglobinopathies. Primer extension labeling of alpha- and beta-thalassemia probes can be accomplished using oligonucleotide pairs such as in the following examples, without dideoxyribonucleotide blockage of the 3' end of the template:

1. homozygous alpha-thalassemia

```
5' ACCAAGACCTACTT
          CTGGATGAAGGGCG 5'
```

2. beta$^{+}$ (IVS-1) thalassemia

```
normal   5' CTGCCTATTGGTC
                    ATAACCAGATAAAA 5'
mutant   5' CTGCCTATTAGTC
                    ATAATCAGATAAAA 5'
```

3. beta° (beta-39) thalassemia

```
normal   5' CCTTGGACCCAGA
                  CCTGGGTCTCCAAGA 5'
mutant   5' CCTTGGACCTAGA
                  CCTGGATCTCCAAGA 5'
```

Using nucleoside triphosphates (NTP) which are to be added to the desired strand, it is possible to separate the starting materials (unreacted) by gel electrophoresis or by column chromatography. Any type of labeled oligonucleotides can be prepared by proper choice of the labeled NTPs. As, for example, with a 19A' pair, if a biotinylated dUTP and dCTP are used substrates, a biotinylated oligonucleotide specific for the normal globin gene can be synthesized; with a fluorescein labeled NTP a fluorescent oligonucleotide can be prepared. It is evident that the particular type of label used, whether a radiolabel or a nonradioisotopic label, is not critical, nor is the method by which the labeled probe is prepared. Further, it is clear that any method for detecting the presence of hybridized probe in the dot-blot can be used, whether employing labeled probes or another technique not involving labeled probes.

The present invention will now be illustrated, but is not intended to be limited, by the following specific examples.

## EXAMPLES

### Example 1: Dot-Blot for Detecting the Sickle Cell Mutation in the Human Hemoglobin Gene

The following solutions are referred to in the examples:

Random Primer buffer (5X)
0.2 M KCl
0.25 M Tris-Cl pH 8.1
10 mM dithiothreitol
25 mM MgCl2

Denhardt's solution (100X)
2% Bovine serum albumin
2% Polyvinylpyrrolidine 40 (Calbiochem, LaJolla, CA, USA)
2% Ficoll (Sigma, St. Louis, MO, USA)
2% sodium dodecylsulfate (SDS)

SSC (20X)
175.3g NaCl
88.2g NaCitrate
pH to 7.0, volume to 1 liter with deionized water

NET (20X)
3M NaCl
0.3 M Tris-Cl pH 7.5
20 mM EDTA

A. Preparation and Labeling of the Oligonucleotide Probes

A primer extension reaction was used to label the oligonucleotide probes with 32-Phosphorous. The radioactive dATP is dried in an Eppendorf tube (1.5 ml) in a Speed-Vac apparatus (Savant, Hicksville, NY, USA). Then the chilled components are added to the tube. For labelling of the normal probe (A') the reaction mix consists of:

0.5 mCi (32-P)-dATP (Amersham, Arlington Heights, IL, USA)
No. PB20474, 6000 Ci/mMole)
1 µl (200 ng) DNA primer (5'-GCAGACTTCTCCTC)
1 µl (400 ng) DNA template (5'-CTCCTGAGGAGAAG)

1 µl 0.13 mM dGTP

2 µl 5X Random Primer Buffer

3.5 µl deionized water

1.5 µl reverse transcriptase (Molecular Genetics Resources Tampa, FL, USA, No. MG101)

For creation of the sickle probe (S'), the primer of sequence 5'-GCAGACTTCTCCAC and the template of sequence 5'-CTCCTGTGGAGAAG are used.

The reaction mix is tapped gently with the finger to mix the components and incubated at 15° C in a water bath for 90 minutes. The final probe sequences are: A' (5'-GCAGACTTCTCCTCAGGAG) and S'(5'-GCAGACTTCTCCACAGGAG). Under hybridization conditions the 14-base pair primer-template duplex dissociates, obviating the need for purification.

Since excess dATP gives a high background binding, it is degraded by addition of 20 µl 0.1 M Tris-Cl, pH 8.7 and 1 µl of calf intestinal alkaline phophatase (Boehringer-Mannheim Indianapolis, IN, USA, No. 108-146, 50 units/µl) and incubation at room temperature for 20 minutes.

An alternative method for removing excess dATP that gives excellent background reduction is the spun column method. A few glass beads of about 0.5 mm diameter are placed in a yellow Eppendorf micropipet tip suspended over a polypropylene test tube. A washer consisting of the upper half of a blue 1 ml Eppendorf micropipet tip is useful for this purpose. The yellow tip is filled with Sephadex G-25 in a TE buffer (10 mM Tris-Cl pH 7.5, 1 mM EDTA) until the settled bed reaches almost to the top of the pipet tip. The test tube containing the mini-column is then spun in a centrifuge at 2000 rpm for 2 minutes. The liquid is removed from the receiving test tube. The

primer extension reaction is diluted with TE buffer to a volume of 40 μl and then applied to the mini-column. The centrifugation is repeated, 2000 rpm for 2 minutes, and the eluate is transferred to an Eppendorf tube for storage. Polyacrylamide gel electrophoresis of the eluate shows almost no residual dATP, while essentially all of the labelled oligonucleotide is recovered.

B. Isolation of Sample DNA

High molecular weight DNA was isolated from peripheral blood lymphocytes or cells present in the amniotic fluid by a method of Blin and Stafford. Nucl. Acid Res. 3,2303-2308 (1976). Briefly, 10-20 ml samples were centrifuged at 3,000 rpm and the plasma or fluid fraction removed by aspiration. Residual amounts of plasma were extracted by repeated washes of the cell pellet with 0.9% NaCl. For DNA extractions from blood samples, the reticulocytes and older red cells were hemolyzed by the addition of two cell pellet volumes of sterile water and the lymphocytes collected by centrifugation at 3,000 rpm for 15 min. The lymphocytes and amniotic fluid cells were lysed by the addition of 20 ml of lysing solution (0.05 M Tris pH 7.5, 0.5% sodium dodecylsulfate (SDS), 0.1 M NaCl, 0.001 M EDTA, 100 μg/ml proteinase k, and incubated at 37°C for 48 hours. Following lysis, the solution was deproteinized by repeated chloroform-phenol (1:3) extractions and dialyzed overnight at 4° C against 50 mM Tris pH 8, 10 mM EDTA, 10 mM NaCl. Nucleic acids were isolated by the addition of 2.5 volumes of ethanol followed by centrifugation at 9,000 rpm for 15 min. The pellet was resuspended in 10 mM Tris pH 7.5, 1 mM EDTA, incubated at 37 degrees C for 30 min. with 50 μg/ml heat-treated RNAse (RNAse A, Sigma, St. Louis, MO, USA), and chloroform-phenol extracted. DNA was

collected by ethanol precipitation followed by centrifugation above, and redissolved in sterile water at a concentration of about 0.2 mg/ml.

C. Immobilization of Sample DNA by Dot-Blotting

Sample DNA is denatured with NaOH, then applied to nitrocellulose paper for hybridization detection. Since hybridization with both the normal and sickle probes is necessary for positive genotype determination, two dots from each patient must be prepared. The concentration of the DNA test samples is determined by diluting 10 µl into 1 ml TE buffer and reading the $OD_{260}$ on a Varian 2200 spectrophotometer Palo Alto, CA, USA. Ten micrograms of test DNA are added to 200 µl 0.5 M NaOH and allowed to denature for 10 minutes at room temperature. Meanwhile, the filter is prepared. BioRad nitrocellulose paper (No. 162-0116 Richmond, CA USA) is cut to approximately 12 X 8 cm and wetted by gradual application to a dish containing 6X SSC buffer. The upper side is then wetted by submersion of the paper. Paper that is not properly wetted will not function consistently. After about 5 minutes of soaking, the paper is applied to a BioBlot filter apparatus (BioRad) and tightened securely. Vacuum is then applied and the screws are retightened.

Immediately before application of the sample, the base is neutralized by addition of 34 µl 2 M ammonium acetate, pH 5.0. Half the test sample is applied to each of two wells on the dot-blot apparatus. If the test sample volume is greater than about 50 µl, all volumes are doubled.

After application of samples, the filter is dried in a vacuum oven at 80° C for 20-60 minutes. It is then prehydridized in a sealed plastic bag for at least ten

minutes at 50° C in the following solution:

50 mM sodium phosphate pH 6.5
5X SSC
5X Denhardt's solution
250 μg/ml sonicated salmon sperm DNA (Sigma)

The filter is then blotted dry with filter paper and cut into strips for hybridization. (At this point the filters can be refrigerated for several weeks.)

D. Hybridization with Labeled Oligonucleotide Probes

For molecular hybridization, the following mixture is prepared:

450 μl 20X NET buffer
750 μl 20% dextran sulfate
150 μl deionized water
75 μl 100X Denhardt's solution
75 μl 10% NP-40 detergent (Sigma)

To this mixture is added a one tenth portion of the oligonucleotide probe prepared in the section above. The final 19-mer probe concentration is approximately 1 nanomolar. It has been found that addition of more probe than this results in an increased background. To prepare labeled probe for a single or a few reactions, the labeling protocol detailed above can be scaled down, using proportionally less primer, template, dGTP, and dATP. A 0.1X reaction can be done in 10 μl, a 0.2X reaction can be done in 20 μl, etc. When volume permits, the [alpha-$^{32}$P]-alpha-ATP need not be evaporated to dryness.

Filter strips are placed in plastic bags (e.g., Seal-A-Meal, Seal and Save, etc.) with one side left open. Up to six different hybridizations can be done in one bag, with sealed partitions between. Several strips can be hybridized per compartment; two are routinely done, back-to-back. The radioactive hybridization mix is added with a Pasteur pipet or a syringe with a 21 gauge needle and the last side of the bag is sealed. Hybridization for less than 30 minutes yields less than maximal hybridization, and a period longer than 1 1/2 hours leads to high background.

After hybridization, excess solution is removed with a syringe fitted with a 21 gauge needle. This mix can be used repeatedly. Strips are recovered by excision with a razor blade and transferred to the cover of a large plastic petri dish (diameter 15 cm) and rinsed briefly with 6X SSC. They are then transferred to the bottom of the petri dish and washed with 6X SSC for 15 minutes with gentle shaking on an orbital shaker.

For stringency washing, the strips are blotted dry and transferred to a prewarmed 15 ml culture tube containing 14 ml 6X SSC. Two strips are washed per tube. The tubes are replaced in a 57° C circulating water bath for 10 minutes. The liquid is poured off, another 15 ml prewarmed 6X SSC is added, and the tubes are replaced in the water bath for another 10 minutes. The tubes are intermittently inverted during the stringency washing. The strips are blotted dry, and allowed to air dry. They are affixed to filter paper with one strip of double sided tape at the top and another at the bottom. The strips are covered with plastic wrap and autoradiographed at -70° C with Kodak XAR-5 film and Cronex Lightning-Plus intensifying screens. An easily readable signal is obtained after overnight exposure.

E. Results

Two known samples (XY as normal control and BG as sickle control) as well as 10 double blind samples were analyzed as above. The figure of the drawing shows the results of the hybridization after 36 hours exposure to X-ray film. Also included are two non-essential positive controls labeled M13A and M13S. These are replicative form M13 virions that contained cloned fragments of normal and sickle DNA, respectively.

As can be seen, a normal signal is comprised of a strong reaction with the normal (A') probe and a weak cross-reaction with the sickle (S') probe. A sickle signal is comprised of no reaction with the normal probe and a strong reaction with the sickle probe. Heterozygotes (samples 5,8,9) are characterized by reaction with both probes, as one would predict. This reaction can easily be distinguished from the normal reaction (samples XY, 4,6,7,10, and 1) by the intensity of the A' and S' dots: normal samples show considerably stronger reaction with the A' probe, while heterozygotes show considerably stronger reaction with the S' probe. Sample 3 has a lower signal than BG or sample 2, the other sickle DNA samples; this is due to a reduced amount of DNA in the applied sample (about 1.5 µg instead of 5 µg). Sample 2 shows also less DNA when analyzed by agarose gel electrophoresis. These cases show that despite variation in DNA amount and purity, correct genotype determination can be easily made. In addition, because the procedure is so rapid, repeat determinations can be conveniently made.

These test samples were received by mail and the assay was performed. Several individuals read the film and arrived at the same diagnosis. The genotypes, which had been determined by Southern blot analysis (RFLP) in the lab where samples were purified, were then obtained by

telephone. The blind diagnoses were correct in 100% of the cases. This was repeated on two occasions with 38 other unknown samples, with the same result.

Example 2: Probes For Detection of Sickle Mutation

For the detection of the sickle mutation the following three probes were synthesized, by using the phosphoramidite method and an applied biosystem model 380B DNA synthesizer:

(1) $^{5'}$GATGATGCGATGATCC$^{3'}$.....(16L)

(2) $^{5'}$GGATCATCGCATCATCGCCCGGCAGACTTCTCCTCAGGAGT$^{3'}$......(41A')

(3) $^{5'}$GGATCATCGCATCATCGCCCGGCAGACTTCTCCACAGGAGT$^{3'}$......(41S')

The labeling reactions were carried out by mixing ten micrograms of the small probe 16L and twenty micrograms of the long probe 41A' or 41S' dissolved in 12.1 microliter of buffer. To this, 60 micrograms of aminoethyltrioxsalen PEG biotin compound AMT-PEG-biotin (structure of which is given hereinbelow) was added and the final volume was adjusted to 160 microliter with 10mM sodium borate, pH 8. Then the total mixture was split into four tubes and irradiated from above on ice for 60 minutes with a hand held UV lamp model UVL-56 "BLAK-RAY". After irradiation, the labeled oligonucleotides were separated from the unlabeled materials and the starting reagents by gel electrophoresis. A 15% polyaoylamide gel containing 7M-urea was run for 15 minutes at 250 volts. The dimensions of the gel were 14cm long, 19cm wide, and 1.5 mm thick. The gel showed three bands under UV shadowing (the gel was placed on a fluorescent screen and observed with UV light.) The slowest moving band was taken as the cross-linked oligonucleotide, as the other ones comigrated with unlabeled oligonucleotide standards. The three sets of bands were then cut out from the gel, placed in 1.5 ml Eppendorf tubes, and extracted with 1 ml of 20X SSC (20X SSC is 3.0M/NaCl/.3M/NaCitrate). The gel mixture was

vortexed and incubated at 37°C for 16 hours on a gentle mechanical shaker. To purify the probes further, the gel was removed by centrifugation. The pellet was washed once with distilled water, and the pooled supernatants were applied to Nensorb columns (DuPont). Washing and elution in methanol/water were done as described by the manufacturer. Fractions were then evaporated to dryness under vacuum in a Savant Speed-vac apparatus.

Test for biotin: Different gel fractions were tested for biotin by diluting 2 microliters of the sample into 23 µl of water and spotting 1 microliter of this onto a nitrocellulose paper (BioRad). Filters were air dried overnight and were baked in a vacuum oven at 80°C for 30 minutes. The filters were pre-hybridized for 90 minutes at 50°C in blotto, which is 2.5 gm of Carnation non-fat dry milk, 15 ml 20X SSC, and 5 ml, 0.2M sodium pyrophosphate adjusted to 50 ml with water. Filters were then detected with a BRL kit including streptavidin and polyalkaline phosphatase according to the manufacturer's specifications. To test for the hybridizability of such probe, M13 phage DNA containing an insert of either the normal or sickle beta-globin gene were spotted and hybridized as described in the example. After hybridization and washing at 64°C they were detected by the BRL (Betheseda Research Laboratory, Betheseda, Maryland, U.S.A.) protocol. The results indicate that the probes prepared this way show both hybridizability and specificity.

Fig. 2 shows a M13 replicative form viroid containing a cloned fragment of normal (A) or sickle (s) human beta-globin that was dot-blotted as described. Nanogram amounts applied to the spots are indicated along the left side. The top four dots contain the normal (A) sequence; the second set of four contain the sickle (S)

sequence. The C dot is a positive control consisting of 200 pg biotinylated lambda DNA supplied as part of the DNA detection kit (BRL). The left strip was hybridized with normal specific prolic (A'), and the right strip with sickle specific probe (S') as described herein.

Synthesis of AMT-PEG-Biotin:

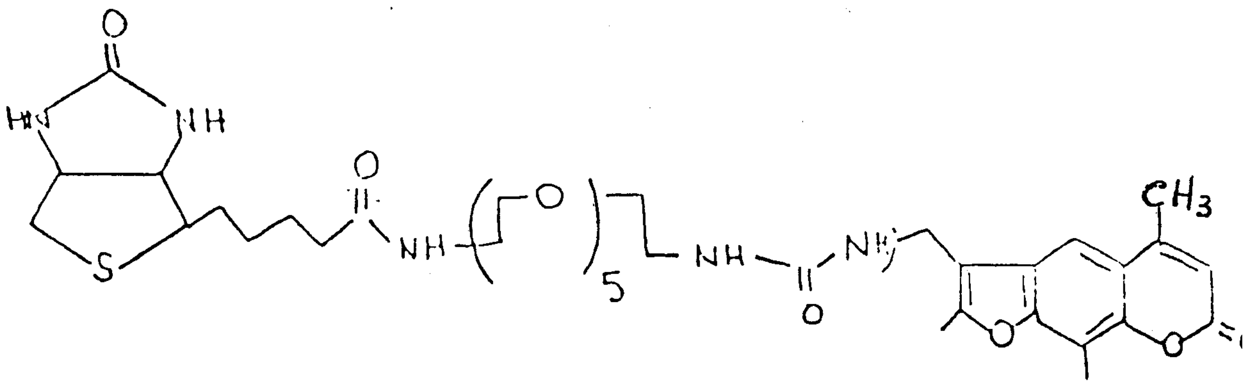

AMT-PEG-Biotin

The preparation of AMT-PEG-Biotin required 1-amino-17-N-(Biotinylamido)-3,6,9,12,15 pentaoxaheptadecane. This was achieved in four stages, namely as follows:

(1) 3,6,9,12,15 pentaoxaheptadecane (x)-1,17-diol ditosylate was synthesized.

(2) 1,17-dipthalimido derivative of (x) was prepared.

(3) 1,17-diamino derivative of (x) was prepared.

(4) 1 amino, 17-biotinylamido derivative of (x) was prepared.

Preparation of 3,6,9,12,15-Pentaoxaheptadecane-17,diol-ditosylate:

To a stirred solution containing 50 g of hexaethylene glycol (0.177 mol) and 64 ml of triethylamine (39.36 g,

0.389 mol) in 400 ml of $CH_2Cl_2$ at 0°C was added dropwise a solution containing 73.91 g of p-toluenesulfonyl chloride (0.389 mol) in 400 ml of $CH_2Cl_2$ over a 2.5 hour period. The reaction mixture was then stirred for 1 hour at 0°C and then allowed to warm to ambient temperature for 44 hours. The mixture was then filtered and the filtrate concentrated in vacuo. The resulting heterogeneous residue was suspended in 500 ml of ethyl acetate and filtered. The filtrate was then concentrated in vacuo to a yellow oil which was triturated eight times with 250 ml portions of warm hexane to remove unreacted p-toluenesulfonyl chloride. The resulting oil was then concentrated under high vacuum to give 108.12 g of a yellow oil (quantitative yield).

Analysis: Calculated for $C_{26}H_{38}O_{11}S_2$

Calculated: C, 52.87; H, 6.48

Found : C, 52.56; H. 6.39.

<u>Preparation of 1,17-Diphthalimido-3,6,9,12,15-pentaoxa-hepta decane</u>:

A stirred suspension containing 108 g of 3,6,9,12,15-pentaoxaheptadecane-17-diol ditosylate (0.183 mol), 74.57 g of potassium phthalimide (0.403 mol), and 700 ml of dimethylacetamide was heated at 160-170°C for 2 hours and was then allowed to cool to room temperature. The precipitate was filtered and washed with water and acetone to give 53.05 g of product as a white powder which was dried at 55°C (0.1 mm). mp 125-126°C.

A second crop of product was obtained from the dimethylacetamide filtrate by evaporation in vacuo and successively washing the resulting precipitate with ethyl acetate, water, and acetone. The resulting white powder was dried at 55°C (0.1 mm) to give an additional 9.7 g of product. mp 125.4-126.5°. The combined yield of product was 62.82 g (68% yield).

Analysis: (For first crop)

Calculated for $C_{28}H_{32}N_2O_9.1/2H_2O$

Calculated: C, 61.19; H, 6.05; N, 5.09

Found : C, 61.08; H, 6.15; N, 5.05.

(For second crop)

Calculated for $C_{28}H_{32}N_2O_9$

Calculated: C, 62.21; H, 5.97; N, 5.18

Found : C, 61.78; H, 6.15; N, 5.13.

Preparation of 1,17-Diamino-3,6,9,12,15-Pentaoxaheptadecane:

A solution containing 60 g of 1,17-diphthalimido-3,6,9,12,15-pentaoxaheptadecane (0.118 mol), 14.8 g of hydrazine hydrate (0.296 mol) and 500 ml of ethanol were heated with mechanical stirring in a 100°C oil bath for 3 hours. The mixture was then allowed to cool and was then filtered. The filter cake was washed four times with 300 ml portions of ethanol. The combined filtrates were concentrated to give 32.35 g of a yellow opaque glassy oil. Evaporative distillation at 150-200°C (0.01 mm) gave 22.82 g of a light yellow oil (69% yield).

Analysis: For $C_{12}H_{28}N_2O_5.1/2\ H_2O$

Calculated: C, 49.80; H, 10.10; N, 9.68.

Found : C, 50.36; H, 9.58; N, 9.38.

(W. Kern, S. Iwabachi, H. Sato, and V. Bohmer, Makrol. Chem., 180, 2539 (1979)).

Preparation of 1-Amino-17-N-(Biotinylamido)-3,6,9,12,15-pentaoxaheptadecane:

A solution containing 7.2 g of 1,17-diamino-3,6,9,12,15-pentaoxaheptadecane (25 mmol) in 75 ml of DMF under an argon atmosphere was treated with 3.41 g of N-succinimidyl biotin (10 mmol) added in portions over 1.0 hour. The resulting solution was stirred for 4 hours at ambient temperature. TLC ($SiO_2$, 70:10.1 $CHCl_3$-$CH_3OH$-conc.

$NH_4OH$) visualized by dimethylaminocinnamaldehyde spray reagent showed excellent conversion to a new product (Rf=0.18). The reaction mixture was divided in half and each half was absorbed onto $SiO_2$ and flash-chromatographed on 500 g of $SiO_2$-60 (230=400 mesh) using a 70:10.1 $CHCL_3$-$CH_3OH$-conc. $NH_4OH$ solvent mixture. Fractions containing the product were pooled and concentrated to give 2.42 g of a gelatinous, waxy solid. The product was precipitated as a solid from isopropanol-ether, washed with hexane, and dried at 55°C (0.1 mm) to give 1.761 g of a white powder (35% yield).

Analysis: Calculated for $C_{22}H_{42}N_4O_7S.3/2\ H_2O$:

Calculated: C, 49.51; H, 8.50; N, 10.49.

Found : C, 49.59; H, 8.13; N, 10.39.

<u>Preparation of 4'-(Biotinyl-PEG)-Trioxsalen (AMT-PEG-Biotin)</u>

A solution of 380 mg of 1-Amino-17-N-(Biotinylamido)-3,6,9,12,15-pentaoxaheptadecane (0.75 mmol) in 3 ml of DMF under an argon atmosphere was treated with 146 mg of N,N-carbonyldiimidazole (0.9 mmol). The resulting solution was stirred for 2.5 hours. TLC ($SiO_2$, 4:1 $CHCL_3$-$CH_3$)OH, visualization with dimethylaminocinnamaldehyde spray reagent) indicated a complete conversion of biotinylamine (Rf=0.1) to imidazourea (Rf=0.5). The reaction mixture was then treated with 193 mg of 4'-aminomethyl-4,5',8-trimethyl-psoralen (commercially available from HRI, California, U.S.A.) (0.75 mmol) and 2.7 µl of triethylamine (1.57 mmol). The resulting mixture was then heated at 60°C overnight. TLC ($SiO_2$, 4:1 $CHCl_3$-$CH_3OH$) indicated conversion of imidazolide to a new product (Rf=0.52) which is both uv fluorescent and tested positive with the dimethylamino-cinnamaldehyde spray reagent. The solvents were removed in vacuo to a gelatinous oil, which was dissolved in $CH_3OH$ and absorbed

onto $SiO_2$. The impregnated solid was then flash-chromatographed on 60 g of $SiO_2$-60 (230-400 mesh) using a 9:1 $CHCl_3$-$CH_3OH$ solvent mixture. Fractions containing the partially purified product were pooled and then rechromatographed using 60 g of $SiO_2$ eluted with the same solvent system.

mp: decomposed slowly 129.5°C to 149.5°C.

Analysis: Calculated for $C_{38}H_{55}N_5O_{11}S.H_2O$:

Calculated: C, 56.49, H, 7.11; N, 8.67

Found : C, 56,58; H, 7,16; N, 8.53.

The above examples and description provide representative embodiments of the present invention. Obviously many other modifications can be made without departing from the inventive features of the present method.

WHAT IS CLAIMED IS:

1. A method for detecting a particular base sequence in a sample of eucaryotic genomic DNA by nucleic acid hybridization, comprising the steps of:

(a) immobilizing DNA from the sample in single stranded form in a limited zone of a solid support sheet,

(b) treating the support sheet to substantially block unoccupied nucleic acid binding sites thereon,

(c) incubating the support with a solution containing an oligonucleotide probe under hybridizing conditions, such probe having a base sequence exactly complementary to the base sequence of interest in the sample,

(d) separating the excess probe solution from the support,

(e) washing the support at a stringency sufficient to remove substantially all probe which had hybridized to sequences other than the exactly complementary sequence of interest, and

(f) detecting the presence of hybridized probe in said zone of the support sheet.

2. The method of Claim 1 wherein the support to which the single stranded sample DNA is immobilized is incubated with the oligonucleotide probe for less than about 4 hours.

3. The method of any of claims 1 and 2 wherein the concentration of the probe in the solution incubated with the support to which the single stranded sample DNA is immobilized is between about 10 pM and 10 nM.

4. The method of any of claims 1 to 3 wherein the sample is a liquid mixture, is treated to denature DNA therein and applied to the solid support sheet, and residual liquid removed by vacuum or the sample is applied to the solid support sheet and DNA therein is denatured in situ.

5. The method of any of claims 1 to 4 wherein the probe comprises a detectable label which is detected in said zone of the support sheet in order to detect the presence of hybridized probe.

6. A method for detecting a particular normal or mutated gene in a human patient by a dot-blot hybridization, comprising the steps of:

(a) obtaining a sample from the patient which contains nucleated cells,

(b) isolating essentially unfractionated genomic DNA from cells contained in such sample,

(c) denaturing DNA in the unfractionated genomic DNA isolate by treatment with base to provide genomic DNA in single stranded form,

(d) neutralizing the base-treated liquid isolate,

(e) applying the neutralized liquid isolate to a solid support sheet in two separate limited zones, the support sheet being capable of immobilizing single stranded DNA, and removing residual liquid by application of a vacuum to the reverse side of the support sheet,

(f) contacting the support sheet for a predetermined time with a prehybridization solution comprising agents which bind to and block unoccupied nucleic acid binding sites on the sheet,

(g) cutting the support sheet into two sections; one comprising the first zone and the other comprising the second zone,

(h) contacting one of the cut sections of the support sheet with a hybridization solution comprising an oligonucleotide probe having a base sequence exactly complementary to the normal sequence of the gene of interest and contacting the other section of the support sheet with a hybridization solution comprising an oligonucleotide probe having a base sequence exactly complementary to the mutated sequence, each of the probes comprising a detectable label,

(i) incubating the respective support sections in contact with the hybridization solutions for less than about four hours at a temperature favorable to hybridization between the probes and their complementary sequences,

(j) removing the hybridization solutions from the sections of the support sheet,

(k) washing the support sheet sections at a stringency sufficient to remove substantially all probe which had hybridized to sequences other than their exactly complementary sequences,

(l) detecting the presence of the probe label in said first and second zones of the cut support sheet sections.

7. The method of claim 6 wherein the normal gene of interest is the human beta-globin gene and its mutated form is that which results is human sickle cell anemia.

8. A probe for the detection of a nucleic acid sequence comprising:

(a) a first synthetic oligonucleotide, said first oligonucleotide including

(i) a hybridizing region specific for the detection of a particular nucleic acid sequence, and

(ii) a plurality of nucleotide residues disposed adjacent to said hybridizing region, and

(b) a second synthetic oligonucleotide, when said first and second oligonucleotides are combined there is a formed a single hybridizing molecule containing a double stranded region and a single stranded hybridizable region, and

(c) a label, said label being attached to the double stranded region.

9. A probe according to claim 8 which further comprises a photoreactive intercalator attached to said double stranded region.

10. A probe according to claim 9, wherein a label is attached to said intercalator.

11. A probe according to any of claims 8 to 10 wherein said label is biotin.

12. A probe according to any of claims 8 to 11 comprising $^{5'}$GATGATGCGATGATCC$^{3'}$...(161)or $^{5'}$GGATCATCGCATCATCGCCC-GGCAGACTTCTCCTCAGGAGT$^{3'}$...(41A') or $^{5'}$GGATCATCGCATCATCGCCCGG-CAGACTTCTCCACAGGAGT$^{3'}$...(41S').

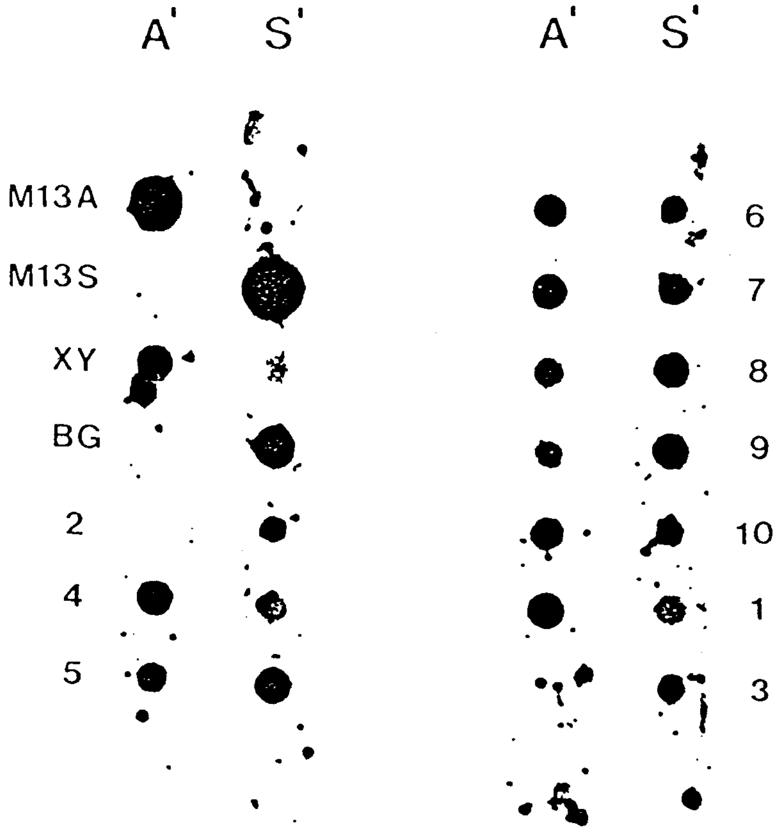


FIG. 1

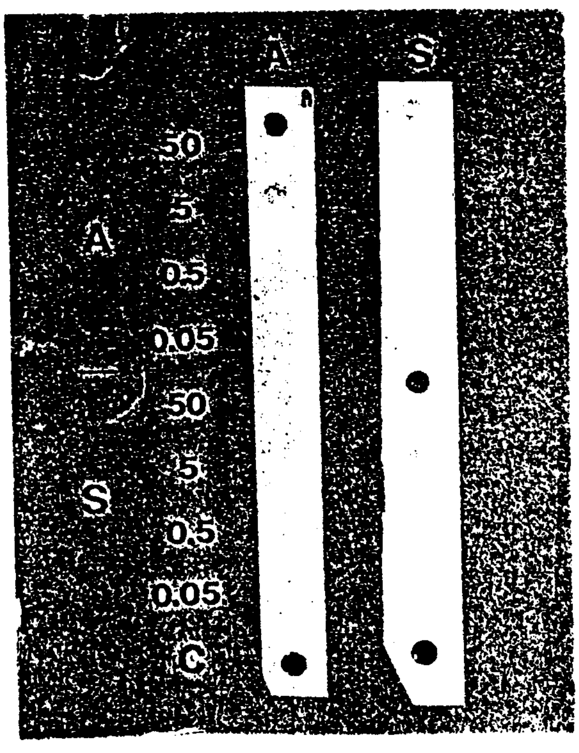


FIG. 2